# EUROPEAN PATENT SPECIFICATION

(11) **EP 1 184 464 B1**
(45) Date of publication and mention of the grant of the patent: **02.11.2005**
(21) Application number: 00935226.1
(22) Date of filing: 06.06.2000
(51) Int. Cl.: C12P 5/02

(54) **LYCOPEN PRODUCTION METHOD**
HERSTELLUNGSVERFAHREN FÜR LYCOPEN
PROCEDE DE PRODUCTION DE LYCOPENE

(30) Priority: 09.06.1999 ES 9901271
(43) Date of publication of application: 06.03.2002
(73) Proprietor: Vitatene, S.A., 24080 Leon (ES)
(72) Inventor: MARCOS, Ana Teresa, E-24080 León (ES); ESTRELLA DE CASTRO, Antonio, E-24080 León (ES); MEHTA, Bina, E-24080 León (ES); DIEZ GARCIA, Bruno, E-24080 León (ES); COSTA PEREZ, Javier, E-24080 León (ES); RODRIGUEZ OTERO, Carmelita, E-24080 León (ES); PEIRO CEZON, Enrique, E-24080 León (ES); CERDA OLMEDO, Enrique, E-24080 León (ES); COLLADOS DE LA VIEJA, Alfonso J., E-24080 León (ES); SALTO MALDONADO, Francisco, E-24080 León (ES); ESTEBAN MORALES, Manuel, E-24080 León (ES); BERNASCONI, Ermanno, E-24080 León (ES)
(74) Representative: Elzaburu Marquez, Alberto
(86) International application number: PCT/ES2000/000202
(87) International publication number: WO 2000/077234

(56) References cited:
- GB-A- 1 034 944
- GB-A- 1 114 043

## Description

### Field of the invention

The present invention relates to a new method for the production of carotenoids, especially lycopene, starting from submerged cultures of mucor fungi of the *Blakeslea* type. A method of fermentation is described by which it is possible to obtain an increase in the production of lycopene, based on the addition of extracts enriched with trisporic acids. The process is carried out starting from wild strains, or mutants of them, in single or mixed culture. Trisporic acids can be added at the start of or during fermentation.

### State of the art

The carotenoids are compounds that occur abundantly in fruits and vegetables, and have been the subject of numerous studies on account of their properties as antioxidants and as vitamin A precursors. Lycopene, in addition to its antioxidant property, prevents cardiovascular diseases and some types of cancer such as prostate cancer and gastrointestinal cancer and is active in the control of growth (Stahl W. and Sies, H. 1996. Arch. Biochem. Biophys. 336 :1-9; Clinton, SK. 1998. Nutr. Rev. 56 :35-51). Owing to their yellow to red coloration, the carotenoids are also used as a food supplement and colorant in margarine, butter, salad oils, soups, and sauces (Ninet L. and Renaut J. 1979. In: Peppler HJ., Perlman D. (eds.) . Microbial Technology, 2nd ed., Vol. 1 Academic Press, NY, p. 529-544).

Lycopene is a carotenoid that is produced as an intermediate in the biosynthetic pathway of β-carotene (GB 1034944 and GB 1114043) and xanthophylls.

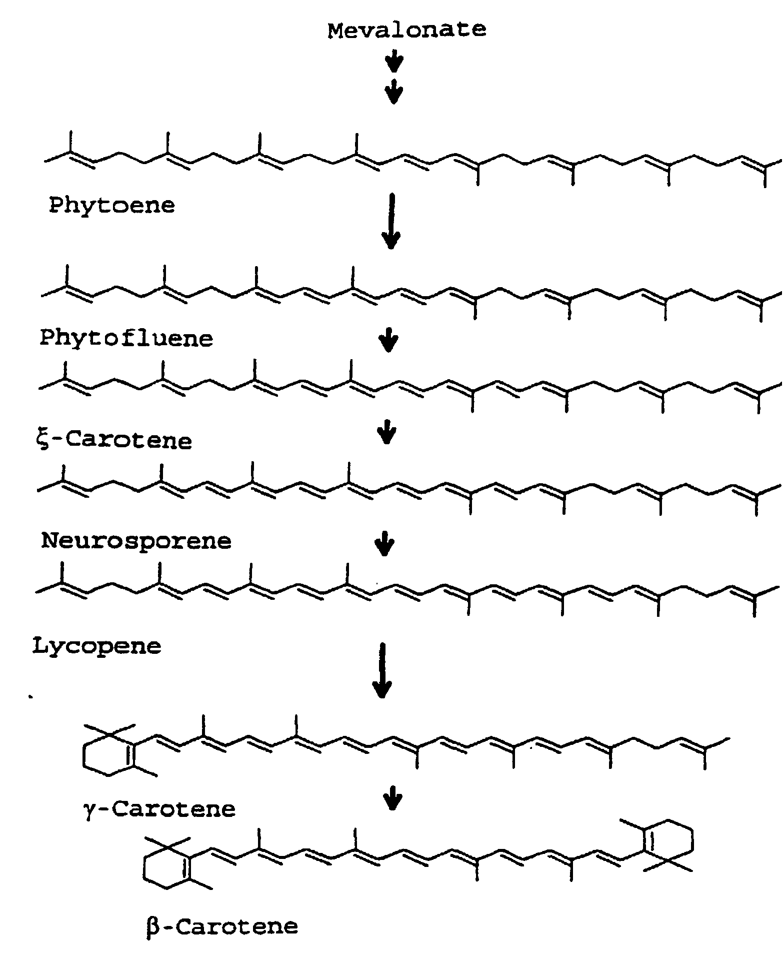

Lycopene preparations are obtained from tomato (Patents PCT WO 97/48287, EP 608027), or by fermentation of mucor fungi such as *Phycomyces, Blakeslea* and *Choanephora* (Patents GB 1008469, US 3097146, US 3369974, JP 73016189, JP 73016190 and RU 2102416).

Patents GB 1008469 and US 3097146 describe a method of fermentation of the fungus *Blakeslea trispora,* consisting of a culture medium whose pH is maintained above 7.0 and up to 9.5. After seven days of incubation, 9.97 mg of lycopene is obtained per 100 ml of medium.

Japanese patents JP 73016189 and JP 73016190 describe a method of fermentation with Mucorales fungi such as *Phycomyces, Blakeslea* and *Choanephora,* to which tertiary amines are added to prevent the formation of β -carotene and promote the accumulation of lycopene. The following are cited as examples of tertiary amines: hordenine, 2-dimethylamino-ethanol; 3-dimethylamino-1-propanol; 1-dimethylamino-2-propanol; 2-diethylaminoethanol; 3-diethylamino-1-propanol; 1-diethylamino-2-propanol; triethylamine; 2-diisopropylamino-ethanol; 3-diisopropylamino-1-propanol; 1-diisopropylamino-2-propanol; 2-di-n-propylamino-ethanol; 3-di-n-propylamino-1-propanol; 1-di-n-propylamino-2-propanol; 2-dimethylaminoethylbenzene; 2-diethylamino-ethylbenzene; p-(2-diethylaminoethyl)phenol. Patent RU 2102416 describes the addition of aminomethylpyridines and tobacco residues to induce the accumulation of lycopene. In addition to the substances described in the said patents, there are descriptions of the use of other nitrogenated heterocyclic bases capable of blocking the synthesis of carotenoids at the lycopene level, such as nicotine (Japanese patent JP 09313167), imidazole, pyridine, morpholine, quinoline and some substituted derivatives (US patent 3369974; Ninet L., Renaut J. 1979. In: Peppler HJ, Perlman D (eds.). Microbial Technology, 2nd Ed., Vol. 1 Academic Press, NY, p. 529-544).

Mutants of *B*. *trispora* that accumulate lycopene without the need to add tertiary amines have also been described (Mehta B. and Cerdá-Olmedo E. 1995. Appl. Microbiol. Biotechnol. 42 :836-838).

In any of the methods of production of β-carotene with the fungus *B. trispora,* mixed cultures of the fungus are used, as a result of which much higher yields of β-carotene are obtained than those encountered in the (+) and (-) strains separately (Ciegler, A. 1965. Advances in Applied Microbiology 7:1-34; Plempel, M. 1965. Planta 65:225-231; Sutter, RP. and Rafelson, ME. 1968. J. Bacteriology 95:426-432). The increase in production of β-carotene in mixed cultures correlates with the production of a family of acidic compounds called factor β or trisporic acids. (Caglioti L., Cainelli G., Camerino B., Mondelli R., Prieto A., Quilico A., Salvatori T., Selva A. 1966. Tetrahedron Supplement 7:175-187). It has been suggested that the trisporic acids are sex hormones in Mucorales fungi such as *B*. *trispora, Phycomyces blakesleanus* and *Mucor mucedo,* for in addition to stimulating the production of β-carotene, they stimulate the formation of zygophores (van den Ende, H. 1968. J. Bacteriology 96:1298-1303; Austin DJ., Bu'Lock JD., Gooday G.W. 1969. Nature 223:1178-1179; Reschke, T. 1969. Tetrahedron Letters 29:3435-3439; van den Ende H., Wiechmann AHCA., Reyngoud DJ., Hendricks T. 1970. J. Bacteriology 101:423-428).

Chemically, the trisporic acids are oxidized, unsaturated derivatives of 1,1,3-trimethyl-2-(3-methyloctyl)cyclohexane. The said compounds can be classified as sesquiterpenes generated or produced in the degradation of diterpenes or carotenoids. The trisporic acids have been characterized as a mixture of three substances: trisporic acids A, B and C, also called factor β₁, β₂ and β₃ (owing to the fact that they stimulate the synthesis of β-carotene). Trisporic acid C is the most abundant, and is found to be present at 80% in mixed cultures of *B. trispora* (Caglioti, L., Cainelli, G., Camerino, B., Mondelli, R., Prieto, A., Quilico, A., Salvatori, T., Selva, A. 1966. Tetrahedron Supplement 7:175-187).

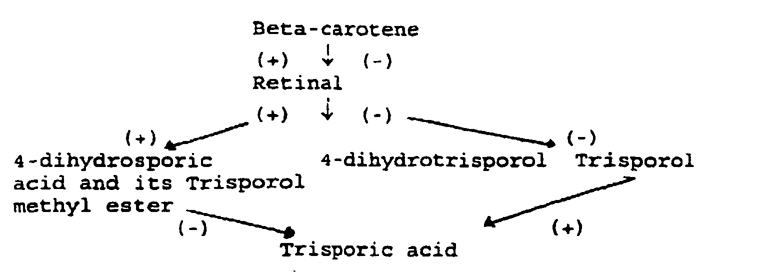

The trisporic acids are synthesized from β-carotene produced both by the (+) strain and by the (-) strain. β-Carotene is metabolized via retinal to 4-hydrotrisporol. This compound is metabolized by the (+) strain to 4-dihydrotrisporic acid and its methyl ester, and the (-) strain converts the 4-hydrotrisporol to trisporol. These two intermediates are converted to trisporic acid only after diffusing in the medium to the strain of the opposite sex (Review by Gooday GW. and Carlile MJ. 1997. Mycologist 11 :26-30). That is to say, the (-) strain produces the intermediates that are converted to trisporic acid by the (+) strain and vice versa, according to the above scheme.

The trisporic acids obtained from mixed cultures of *B. trispora* can be used for increasing the production of β-carotene in cultures with separate strains. For example, addition of 21.8 units of factor β increases by 422% the yield of β-carotene from the (-) strain and by 71% the yield from the (+) strain. However, stimulation of carotenogenesis per unit of factor β in cultures of (-) strain was only 20-30% of carotenogenesis produced in mixed cultures and with similar amounts of factor β. (Sutter RP. and Rafelson ME. 1968. J. Bacteriology 95:426-432).

Trisporic acids can be purified by various methods, such as those described by Sutter, R. 1970. Science 168:1590-1592 or Sutter RP., Capage DA., Harrison TL., Keen WA. 1973. J. Bacteriology 114:1074-1082.

It is not known whether a positive effect of increase in yield of lycopene through the addition of trisporic acids has been described. The production of lycopene is based on suppressing lycopene cyclase activity which converts lycopene to β-carotene, which results in low levels of β-carotene in fermentation. The trisporic acids, which are the sex hormones that induce carotenogenesis, are synthesized from β-carotene. Therefore absence of precursor (β-carotene) is what inhibits the formation of the said hormones and permits increase in production of lycopene by means of trisporic acids, since in mixed cultures that produce β -carotene (as in the case of mutant SB34(-) with ASA2(+) in examples 5 and 6), the addition of trisporic acids has no effect.

### Detailed description of the invention

The object of the present invention claims to increase the production of carotenoids, especially lycopene, in a process of fermentation with Mucorales fungi (*Blakeslea, Choanephora* or *Phycomyces*), more specifically in *B*. *trispora.* The invention consists of cultivating the fungus *B. trispora* in a fermentation medium and adding trisporic acids (or factor β) partially purified (PTA) or even unpurified (NPTA) from some other source, for example from a medium for production of β-carotene.

The effect of the trisporic acids on the fermentation of lycopene is based on the fact that the said hormones are synthesized from β-carotene, a compound that cannot be formed in the fermentation of lycopene. In these conditions the fungus is without a precursor for the synthesis of the said hormones, and carotenogenesis is not induced to its maximum extent.

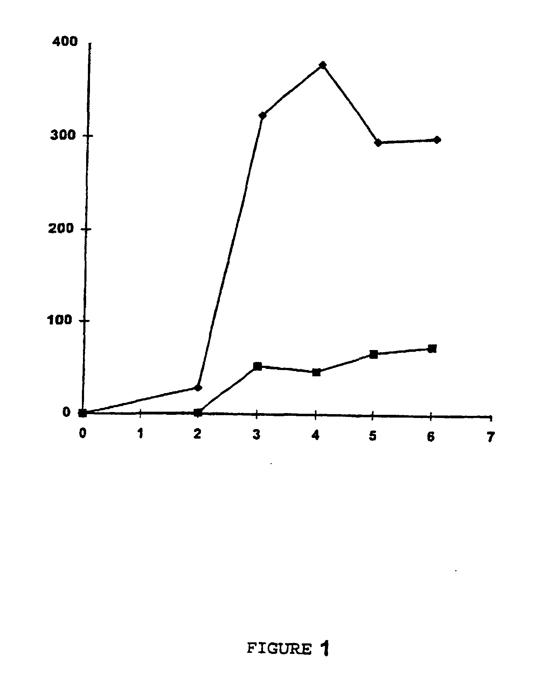

The organisms used for the fermentation can be isolated strains or a mixture of the (+) and (-) strains of Mucorales fungi, more specifically *B*. *trispora,* or alternatively mutants of *B*. *trispora* capable of accumulating lycopene. Various mixtures of (+) and (-) strains of the fungus can be used in the process of fermentation of this invention. When using strains of *B*. *trispora* that are producers of β-carotene, compounds are added that are able to block carotenogenesis at the level of lycopene (for example tertiary amines and others mentioned previously). When using mutants blocked in the biosynthesis of carotenoids from lycopene, it will not be necessary to add tertiary amines and related compounds. In either one of the two cases mentioned, the trisporic acids are added partially purified (PTA) or unpurified (NPTA) from some source of them, for example from a culture medium derived from fermentation of β-carotene, and in both cases at an approximate concentration of 0.35 mg/ml. The term NPTA corresponds to a β-carotene medium. The concentration of trisporic acids in the said media can be calculated as a function of the day of incubation from Fig. 1 (on the 4th day there would be 350-400 µg/ml).

The process for the production of carotenoids from Mucorales fungi, and more specifically of lycopene with selected strains or mutants of *B*. *trispora,* can be carried out in any culture medium that contains one or more sources of carbon, one or more sources of nitrogen, mineral salts and thiamine. The sources of carbon can be added as simple or complex nutrients and include carbohydrates or fats, such as dextrins, starches, glucose, saccharose, fructose, animal or vegetable oils. The culture medium must also have an assimilable source of nitrogen, organic or inorganic, such as soya flour, maize flour, soluble distillates, yeast extract, cottonseed flour, peptone, casein, ammonium sulphate etc. The mineral salts that can be added to the culture medium include phosphates, sulphates, chlorides, sodium, potassium, ammonium, calcium, and magnesium. The proportions of the nutrients are determined on the basis of the requirements for growth of the microorganism and on the levels of production. Fermentation is preferably carried out in aerobic conditions and in submerged culture. The fermentation temperature can vary between 20 and 32°C, though between 25 and 28°C, is preferred.

Addition of trisporic acids in a concentration ranging 0.001-1000 mg/ml can be effected from a partially purified preparation or from any source of them, for example culture medium derived from fermentation of β-carotene of any mucor fungus. Addition is preferably effected between 72-144h from the start of fermentation.

The lycopene is extracted by any method that has been described for cell rupture that makes it possible to release the cell contents, which are dissolved in any solvent and in which they are soluble. Its concentration can be determined by measurement by spectrophotometry, but the use of liquid chromatography (HPLC) is preferred, by any of the methods describes in the bibliography.

The strains used are as follows: Mutants that are producers of β-carotene: ASA2(+) and ASA25(-). Mutant that is a producer of lycopene SB34(-). This last-mentioned mutant also produces 25% of β-carotene (see Table 1). Selection of the mutants is irrelevant from the point of view of the effect of increase in carotene production due to the trisporic acids per se. That is, the effect due to the said compounds would be reproducible in any other similar mutant strain, although it would have a lower basal production of lycopene, as occurs with parent strains, for example. The parent strains are deposited in a collection and are viable and are available to the public.

### Example 1.

### Determination of the concentration of trisporic acids throughout fermentation of β-carotene and lycopene.

An inoculating medium is prepared containing, per litre: soya flour, 23 g; maize flour,47 g; monopotassium phosphate 0.5 g; thiamine hydrochloride, 0.002 g. Its initial pH is 6.3. The medium is distributed in 500 ml Erlenmeyer flasks at a rate of 67 or 100 ml. After sterilizing, the strains *B*. *trispora* ASA2(+) and *B. trispora* ASA25(-), from suspensions of spores, are sown in separate flasks and are incubated at 25°C for 48 hours.

The mutant strains *B*. *trispora* ASA2(+) and *B*. *trispora* ASA25(-) are obtained by mutation with NTG from the wild strains *B. trispora* F208(-) and F117(+) deposited in the Collection of industrial Microorganisms (VKM), located in Moscow, Russia, both strains being available to the public.

A basic fermentation medium is prepared containing, per litre: soya flour, 44 g; maize flour, 19 g; dibasic phosphate 0.55 g; thiamine hydrochloride, 0.002 g; vegetable oil 10%. Its initial pH is adjusted to 7.5 with potassium hydroxide. The medium is distributed in 250 ml Erlenmeyer flasks at the rate of 20 ml.

For the fermentation of β-carotene, flasks are inoculated that contain basic medium with 10% of a mixed culture of the strains *B*. *trispora* ASA2(+) and *B*. *trispora* ASA25(-). For the fermentation of lycopene, a chemical agent is added to the basic medium with the aim of blocking the biosynthetic pathway at the level of lycopene, for example 2 ml/l of pyridine. This medium is inoculated with 10% of a mixed culture of the strains *B. trispora* ASA2(+) and *B. trispora* ASA25(-). All the flasks are incubated at 25°C in an orbital stirrer at 250 rpm. Starting from the second day of fermentation, a sample is taken every 24 hours and the concentration of trisporic acids is determined in flasks producing β-carotene and in flasks producing lycopene.

The concentration of trisporic acids is determined by extraction with one volume of chloroform after acidifying the sample to pH 2. The organic fraction is extracted with one volume of a 4% solution of sodium bicarbonate, the aqueous phase is collected and is acidified, then extracted with chloroform again. Then the chloroform is evaporated to dryness and the residue consisting of trisporic acids is dissolved in ethanol or Tris-sulphate buffer. The trisporic acids are determined quantitatively by absorption spectrophotometry at 325 nm, assuming an absorptivity of 70 ml x mg⁻¹ x cm⁻¹ (Sutter RP., Capage DA., Harrison TL., Keen WA. 1973. J. Bacteriology 114:1074-1082).

The concentration of trisporic acids was found to vary in the course of fermentation of β-carotene and lycopene, being significantly lower for the latter. The maximum difference was found on the 4th day, with a concentration of 378 µg/ml in the fermentation of β-carotene and of 46 µg/ml in the fermentation of lycopene (an eightfold difference approximately) (Fig. 1) .

### Example 2

### Effect of addition of trisporic acids on the fermentation of a partially blocked mutant in the biosynthesis of β-carotene of the form that accumulates lycopene.

The inoculating medium is prepared as described in Example 1. After sterilizing, the strain *B. trispora* SB34(-) is sown from a suspension of spores and is incubated at 25°C for 48 hours.

The mutant strain *B. trispora* SB34(-) was obtained by mutation with NTG starting from the wild strain *B*. *trispora* F921(-) deposited in the collection of cultures VKM, Moscow, Russia and available to the public.

A fermentation medium is prepared of the same type as described in Example 1. After sterilizing, a series of flasks is inoculated with 10% of the strain *B. trispora* SB34(-), which is characterized in that it is a partially blocked mutant in the biosynthesis of β-carotene of the form that accumulates lycopene. All the flasks are incubated at 25°C in an orbital stirrer at 250 rpm.

The control flasks are those that do not have addition of trisporic acids. The group of flasks that receives addition of trisporic acids is kept in the same conditions as the control until the 4th day, when a preparation enriched with trisporic acids, purified as described in Example 1, is added to a final concentration of 350 µg/ml.

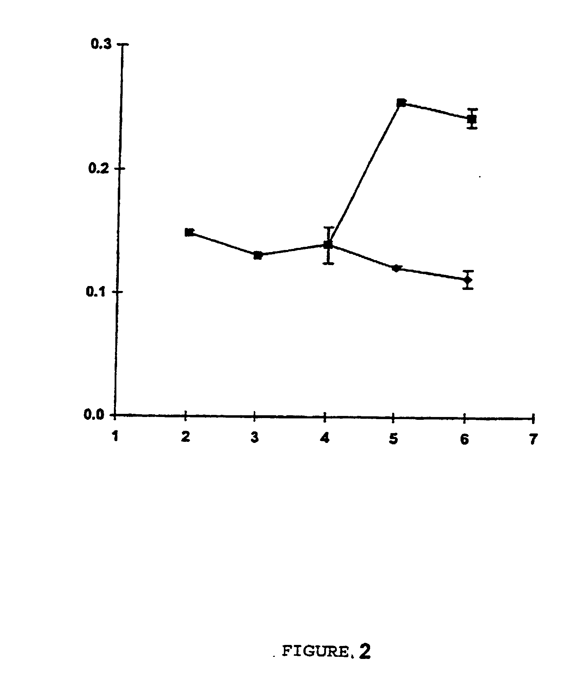

With the addition of partially purified trisporic acids (PTA) we obtain an increase from 0.11 to 0.24 mg of lycopene per gram of dry weight (an increase of 2.18 times relative to the control on the 6th day of fermentation, Fig. 2).

### Example 3

### Effect of addition of trisporic acids on the fermentation of lycopene with a β-carotene-producing mutant whose biosynthesis is blocked at the lycopene level by a chemical agent.

The inoculating medium is prepared in the manner described in Example 1. After sterilizing, the strain *B. trispora* ASA25(-) is sown from a suspension of spores and is incubated at 25°C for 48 hours.

A fermentation medium is prepared of the same type as described in Example 1. After sterilizing, a chemical agent is added which blocks the biosynthesis of carotenoids at the lycopene level, for example imidazole to a final concentration of 0.75 mg/ml. This medium is also supplemented with phosphate buffer pH 7.5 to a final concentration of 25 mM and is inoculated with 10% of the strain *B. trispora* ASA25(-). The flasks are incubated at 25°C in an orbital stirrer at 250 rpm.

The control flasks are those that do not receive addition of trisporic acids. The group of flasks that receives addition of trisporic acids is kept in the same conditions as the control until the 4th day, when a preparation enriched with trisporic acids is added to a final concentration of 350 µg/ml. The trisporic acids are purified in the same way as described in Example 1.

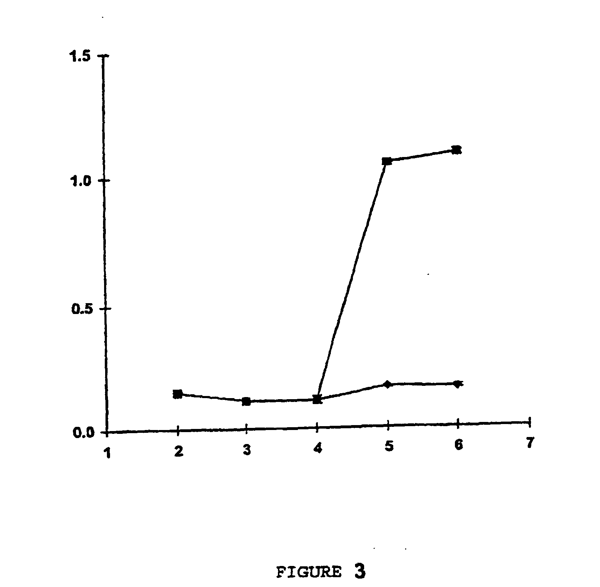

With the addition of partially purified trisporic acids (PTA) we obtain an increase from 0.16 to 1.09 mg of lycopene per gram of dry weight (an increase of 6.8 times relative to the control on the 6th day of fermentation, Fig. 3).

### Example 4

### Effect of addition of partially purified trisporic acids (PTA) or unpurified trisporic acids (NPTA), on the fermentation of the mutant B. trispora ASA25(-) in mixed culture with B. trispora ASA2(+).

The inoculating medium is prepared as described in Example 1. After sterilizing, the strains *B. trispora* ASA25(-) and *B*. *trispora* ASA2 (+) are sown, from suspensions of spores, in separate flasks and are incubated at 25°C for 48 hours.

A fermentation medium is prepared, of the same type as described in Example 1. After sterilizing, imidazole is added to a final concentration of 0.75 mg/ml and phosphate buffer pH 7.5 to a final concentration of 25 mM. This medium is inoculated with 10% of a mixture of the strains *B*. *trispora* ASA25(-) and *B. trispora* ASA2(+). The flasks are incubated at 25°C in an orbital stirrer at 250 rpm.

In order to obtain a source of unpurified trisporic acids (NPTA), a fermentation medium is prepared of the same type as described in Example 1. After sterilizing, a mixed culture is sown with 10% of the strains *B. trispora* ASA2(+) and *B. trispora* ASA25(-), which are producers of β-carotene. The flasks are incubated at 25°C in an orbital stirrer at 250 rpm.

The control flasks are those that do not receive addition of trisporic acids. The group of flasks that receives addition of partially purified trisporic acids (PTA) is kept in the same conditions as the control until the 4th day, when trisporic acids are added to a final concentration of 350 µg/ml. The trisporic acids are purified in the same way as described in Example 1. The group of flasks that receives addition of unpurified trisporic acids (NPTA) is kept in the same conditions as the control until the 4th day, and is then submitted to a process of filtration through a 20 µm nylon filter which makes it possible to separate the mycelium of the strains *B. trispora* ASA25(-) and *B*. *trispora* ASA2(+) from the culture medium where they grew in the presence of imidazole. In parallel, separation of mycelium and culture medium is effected by means of a 20 µm nylon filter, but in this case of flasks that contain the two strains *B. trispora* ASA2(+) and *B. trispora* ASA25(-), in conditions of production of β-carotene, whose culture medium is regarded as an unpurified source of trisporic acids (NPTA). On completion of separation of mycelium and culture medium, the mycelium of the strains *B. trispora* ASA25(-) and ASA2(+) grown with imidazole is mixed with the culture medium of the same strains grown in absence of imidazole. Imidazole is added to the culture medium regarded as an unpurified source of trisporic acids (NPTA), to obtain a final concentration of 0.75 mg/ml. These flasks are incubated at 25°C and 250 rpm.

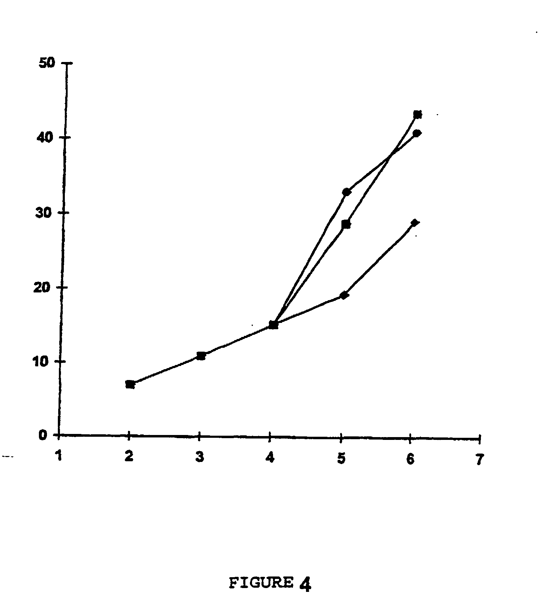

With the addition of partially purified trisporic acids (PTA) we obtain an increase from 29.14 to 43.58 mg of lycopene per gram of dry weight (a 50% increase relative to the control on the 6th day of fermentation, Fig. 3) and by adding culture medium-of β-carotene we obtain an increase from 29.14 to 41.07 mg of lycopene per gram of dry weight (a 41% increase relative to the control on the 6th day of fermentation, Fig. 4). This example shows that the effect of the trisporic acids is independent of their degree of purification.

### Example 5

### Effect of addition of trisporic acids on the fermentation of the mutant B. trispora SB34(-) in mixed culture with B. trispora ASA2(+).

The inoculating medium is prepared in the way described in Example 1. After sterilizing, the strains *B. tri spora* SB34(-) and *B. tri spora* ASA2(+) are sown from suspensions of spores in separate flasks and are incubated at 25°C for 48 hours.

A fermentation medium of the same type as described in Example 1 is prepared. After sterilizing, it is inoculated with 10% of a mixture of the strains *B. trispora* SB34(-) and *B. trispora* ASA2(+). The flasks are incubated at 25°C in an orbital stirrer at 250 rpm.

The control flasks are those that do not receive addition of trisporic acids. The group of flasks that receives addition of trisporic acids is kept in the same conditions as the control until the 4th day, when a preparation enriched with trisporic acids is added to a final concentration of 350 µg/ml. The trisporic acids are purified in the same way as described in Example 1.

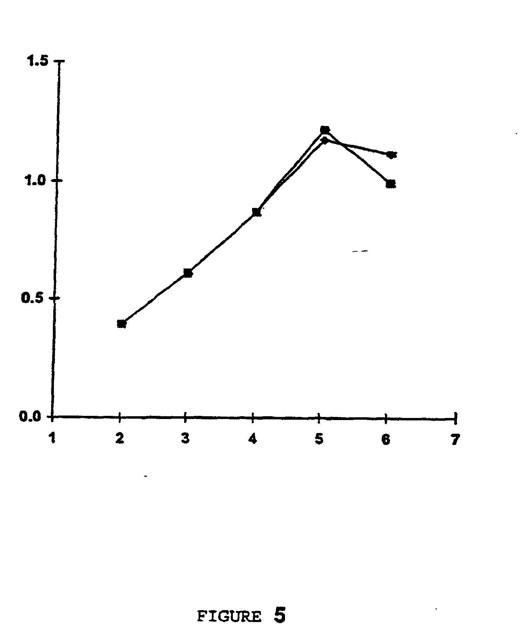

Addition of a preparation enriched with trisporic acids does not have a significant effect on the production of lycopene when fermentation of the mutant SB34(-) is conducted in mixed culture (Fig. 5).

### Example 6

### Analysis of the proportion of carotenoids in mixed fermentations of the strains B. trispora SB34(-) or B. trispora ASA25(-) with B. trispora ASA2(+).

The inoculating medium is prepared as described in Example 1. After sterilizing, the strains *B*. *trispora* SB34(-), *B . trispora* ASA25(-) and *B. trispora* ASA2(+), from suspensions of spores, are sown in separate flasks and are incubated at 25°C for 48 hours.

A fermentation medium of the same type as described in Example 1 is prepared. After sterilizing, this medium is divided into two groups. One group of flasks is inoculated with 10% of a mixture of the strains *B*. *trispora* SB34(-) and *B. trispora* ASA2(+). To the other group of flasks, after sterilizing, imidazole is added to a final concentration of 0.75 mg/ml, and phosphate buffer pH 7.5 to a final concentration of 25 mM. This medium is inoculated with 10% of a mixture of the strains *B. trispora* ASA25(-) and *B. trispora* ASA2(+). All the flasks are incubated at 25°C in an orbital stirrer at 250 rpm.

The control flasks are those that do not have addition of trisporic acids. The group of flasks that receives addition of trisporic acids is kept in the same conditions as the control until the 4th day, when a preparation enriched with trisporic acids is added to a final concentration of 350 µg/ml. The trisporic acids are purified in the same way as described in Example 1.

Samples are taken from the cultures on the 5th and 6th day of fermentation for analysing the proportion of carotenoids by liquid chromatography (HPLC). The results obtained are shown in Table 1.

**Table 1.**

| Percentage of carotenoids on the 5th and 6th day of fermentation in mixed culture of the strains *B*. *trispora* SB34(-) or ASA25(-) with *B. trispora* ASA2 (+) | | | | | | |
|---|---|---|---|---|---|---|
| Strains | Trisporic acids | Day | Neurosporene (%) | Lycopene (%) | γ-Carotene (%) | β-Carotene (%) |
| SB34(-)xASA2(+) | No | 5th | 3 | 57 | 16 | 25 |
| SB34 (-) xASA2 (+) | No | 6th | 3 | 64 | 12 | 21 |
| SB34(-)xASA2(+) | Yes | 5th | 3 | 55 | 15 | 28 |
| SB34(-)xASA2(+) | Yes | 6th | 2 | 62 | 12 | 23 |
| ASA25(-)xASA2(+) | No | 5th | 1 | 88 | 9 | 2 |
| ASA25(-)xASA2(+) | No | 6th | 1 | 89 | 8 | 2 |
| ASA25(-)xASA2(+) | Yes | 5th | 1 | 85 | 10 | 3 |
| ASA25(-)xASA2(+) | Yes | 6th | 1 | 88 | 9 | 2 |

Analysis of the percentage of carotenoids obtained in the fermentation of lycopene using a mixed culture of the strains *B*. *trispora* SB34(-) or *B. trispora* ASA25(-) with *B*. *trispora* ASA2(+) shows a significant difference in the percentage of lycopene and β-carotene (Table 1). These differences are independent of addition of trisporic acids. The mixture of carotenoids obtained on the 6th day by fermentation with *B*. *trispora* SB34(-) has 63% of lycopene, whereas that obtained with *B. trispora* ASA25(-) has 88% (giving a product that is 25% purer). In the same mixture, the proportion of β-carotene is 10 times greater in fermentation with *B. trispora* SB34(-) than with *B*. *trispora* ASA25(-).

As β-carotene is the precursor of the trisporic acids, these data could explain:
1. The difference in concentration of trisporic acids between fermentation of β-carotene and fermentation of lycopene (Example 1) .
2. The fact that addition of trisporic acids (purified or unpurified) gives an increase between 40 and 50% in yield of lycopene on the 6th day of fermentation in mixed culture when *B. trispora* ASA25(-) is used as negative strain, whose mixture of carotenoids contains just 2% of β-carotene (Examples 4 and 6).
3. The fact that addition of trisporic acids has no effect on the mixed culture when *B. trispora* SB34(-) is used as negative strain, whose mixture of carotenoids contains more than 20% of β-carotene (Examples 5 and 6).

These results demonstrate that the effect of the trisporic acids on the fermentation of lycopene in mixed cultures is due to the absence of precursor for its biosynthesis. That is why its use in the industrial production of lycopene of high purity is highly advantageous.

### Detailed description of the diagrams

**Fig. 1**. Concentration of trisporic acids in the course of fermentation of β-carotene and fermentation of lycopene. A mixed culture of the strains *B. trispora* ASA25(-) and *B. trispora* ASA2(+) is used in both fermentations. In the fermentation of lycopene (squares), a chemical agent that promotes the accumulation of lycopene, in this case pyridine, is added to the culture medium. The fermentation of β-carotene (diamonds) is carried out in the same conditions but without pyridine. Abscissa: time in days. Ordinate: concentration of trisporic acids (µg/ml).

**Fig. 2.** Production of lycopene by the mutant strain *B*. *trispora* SB34(-), blocked in the synthesis of β-carotene at the lycopene level, with and without addition of trisporic acids. The values of lycopene yield obtained in a control fermentation without addition of trisporic acids are shown by diamonds. The effect of addition of partially purified trisporic acids (PTA) on the 4th day of fermentation on the yield of lycopene is indicated with squares. The values show the average of two independent samples. The error bars represent the standard deviation from the mean. Abscissa: time in days. Ordinate: mg lycopene/g dry weight.

**Fig. 3.** Production of lycopene by the mutant strain *B*. *trispora* ASA25(-), overproducer of β-carotene, with and without addition of trisporic acids. The fermentation medium is supplemented with imidazole to promote the accumulation of lycopene. The values of lycopene yield obtained in a control fermentation without addition of trisporic acids are represented by diamonds. The effect of addition of partially purified trisporic acids (PTA) on the 4th day of fermentation on the yield of lycopene is indicated with squares. The values represent the average of two independent samples. The error bars represent the standard deviation from the mean. Abscissa: time in days. Ordinate: mg lycopene/g dry weight.

**Fig. 4.** Production of lycopene in mixed culture of the mutant strains, overproducers of β-carotene, *B. tri spora* ASA25(-) and *B. trispora* ASA2(+), with and without addition of trisporic acids. The fermentation medium is supplemented with imidazole to promote the accumulation of lycopene. The values of lycopene yield obtained in a control fermentation without addition of trisporic acids are represented by diamonds. The effect of addition of partially purified trisporic acids (PTA) on the 4th day of fermentation on the yield of lycopene is indicated with squares. The yield of lycopene obtained by addition of unpurified trisporic acids (NPTA) on the 4th day of fermentation is represented by circles. The values represent the average of two independent samples. The error bars represent the standard deviation from the mean. Abscissa: time in days. Ordinate: mg lycopene/g dry weight.

**Fig. 5.** Production of lycopene in mixed culture of the strains *B. trispora* SB34(-) and *B.* trispora ASA2(+), with and without addition of trisporic acids. The values of lycopene yield obtained in a control fermentation without addition of trisporic acids are represented by diamonds. The effect of addition of partially purified trisporic acids (PTA) on the 4th day of fermentation on the yield of lycopene is indicated with squares. The values represent the average of two independent samples. The error bars represent the standard deviation from the mean. Abscissa: time in days. Ordinate: mg lycopene/g dry weight.

## Claims

1. Method of production of lycopene by fermentation of (+) and (-) strains of Mucorales fungi cultures in inhibitory conditions for producing β-carotene, **characterized in that** trisporic acids are added at any moment during fermentation and the lycopene produced is recovered.

2. Method according to claim 1, **characterized in that** the Mucorales fungus used in the fermentation is *Blakeslea trispora*.

3. Method according to any of the claims 1 and 2, **characterized in that** a culture is used that contains a mutant strain of *B. trispora* whose capacity for biosynthesis of β-carotene is completely or partially blocked, owing to the mutation, and which accumulates lycopene.

4. Method according to any of the claims 1 and 2, **characterized in that** an inhibitor of the biosynthesis of β-carotene is added to the culture at any moment in the fermentation, in order to induce the accumulation of lycopene.

5. Method according to any of the preceding claims, **characterized in that** addition of trisporic acids is carried out in such a way that their concentration obtained varies between 0.001 and 1000 mg/ml.

6. Method according to any of the preceding claims, **characterized in that** addition of trisporic acids to the culture is effected between 72 and 144 h from the start of fermentation.

7. Method according to any of the preceding claims, **characterized in that** the trisporic acids added are partially purified.

8. Method according to any of the claims 1 to 6, **characterized in that** the trisporic acids are added without purification, preferably from a culture medium recovered from fermentation of β-carotene.

## Patentansprüche

1. Methode für die Herstellung von Lycopen durch Gärung von (+) und (-)Stämmen von Mucoralespilzkulturen unter Bedingungen, die die Produktion von β-Karoten verhindern, **dadurch gekennzeichnet, daß** irgendwann während der Gärung Dreisporensäuren hinzugegeben werden und das Lycopen zurückgewonnen wird.

2. Methode entsprechend Anspruch 1, **dadurch gekennzeichnet, daß** es sich bei dem für die Gärung benutzten Mucoralespilz um *Blakeslea trispora* handelt.

3. Methode entsprechend irgendeinem der Ansprüche 1 und 2, **dadurch gekennzeichnet, daß** eine Kultur verwandt wird, die einen mutierenden Stamm von *B. trispora* enthält, dessen β-Karotenbiosynthesekapazität wegen der Mutation vollkommen oder teilweise blockiert ist und Lycopen anhäuft.

4. Methode entsprechend einem der Ansprüche 1 und 2, **dadurch gekennzeichnet, daß** irgendwann während der Gärung ein β-Karotenbiosyntheseverzögerer zu der Kultur gegeben wird, um die Anhäufung von Lycopen anzuregen.

5. Methode entsprechend irgendeinem der vorstehenden Ansprüche, **dadurch gekennzeichnet, daß** das Hinzufügen von Dreisporensäuren so durchgeführt wird, daß ihre erreichte Konzentration zwischen 0.001 und 1000 mg/ml schwankt.

6. Methode entsprechend irgendeinem der vorstehenden Ansprüche, **dadurch gekennzeichnet, daß** die Zugabe der Dreisporensäuren 72 bis 144 Stunden nach dem Gärungsbeginn erfolgt.

7. Methode entsprechend irgendeinem der vorstehenden Ansprüche, **dadurch gekennzeichnet, daß** die hinzugegebenen Dreisporensäuren teilweise gereinigt sind.

8. Methode entsprechend irgendeinem der Ansprüche 1 bis 6, **dadurch gekennzeichnet, daß** die Dreisporensäuren ungereinigt, vorzugsweise aus einem von einer β-Karotengärung wiedergewonnenen Kulturmittel, hinzugefügt werden.

## Revendications

1. Méthode de production de lycopène par fermentation de souches (+) et (-) de cultures de champignons Mucorales dans des conditions d'inhibition pour produire du β-carotène, **caractérisée par le fait que** les acides trisporiques sont ajoutés à tout moment pendant la fermentation et que le lycopène produit est récupéré.

2. Méthode suivant la revendication 1, **caractérisée par le fait que** le champignon Mucorales utilisé dans la fermentation est *Blakeslea trispora*.

3. Méthode suivant l'une quelconque des revendications 1 et 2, **caractérisée par le fait qu'**une culture est utilisée qui contient une souche de *B. trispora* dont la capacité de biosynthèse de β- carotène est complètement ou partiellement bloquée, en raison de la mutation, et qui accumule du lycopène.

4. Méthode suivant l'une quelconque des revendications 1 et 2, **caractérisée par le fait qu'**un inhibiteur de la biosynthèse de β-carotène est ajouté à la culture à tout moment dans la fermentation, afin d'induire l'accumulation de lycopène.

5. Méthode suivant l'une quelconque des revendications précédentes, **caractérisée par le fait que** l'addition d'acides trisporiques est effectuée de telle façon que leur concentration obtenue varie entre 0.001 et 1000 mg/ml.

6. Méthode suivant l'une quelconque des revendications précédentes, **caractérisée par le fait que** l'addition d'acides trisporiques à la culture est effectuée entre 72 et 144 h depuis le début de la fermentation.

7. Méthode suivant l'une quelconque des revendications précédentes, **caractérisée par le fait que** les acides trisporiques ajoutés sont partiellement purifiés.

8. Méthode suivant l'une quelconque des revendications de 1 à 6, **caractérisée par le fait que** les acides trisporiques sont ajoutés sans purification, de préférence d'un bouillon de culture récupéré de la fermentation de β-carotène.
